# EUROPÄISCHE PATENTSCHRIFT

(11) **EP 0 900 131 B1**
(45) Veröffentlichungstag und Bekanntmachung des Hinweises auf die Patenterteilung: **09.10.2002**
(21) Anmeldenummer: 96914934.3
(22) Anmeldetag: 18.04.1996
(51) Int. Cl.: B21D 15/10, B29C 49/00, A61B 17/00

(54) **VERFAHREN ZUR OBERFLÄCHENSCHÖNENDEN, VERSTEIFENDEN STRUKTURIERUNG DÜNNER MATERIALBAHNEN**
STRUCTURING PROCESS THAT STIFFENS AND EMBELLISHES THE SURFACE OF THIN MATERIAL WEBS
PROCEDE DE STRUCTURATION RIGIDIFIANT ET EMBELLISANT LA SURFACE DES BANDES DE MATERIAU MINCES

(43) Veröffentlichungstag der Anmeldung: 10.03.1999
(73) Patentinhaber: Dr. Mirtsch GmbH, 14513 Teltow (DE)
(72) Erfinder: MIRTSCH, Frank, D-14513 Teltow (DE); BÜTTNER, Olaf, D-10789 Berlin (DE); MATSCHINER, Frank, D-06114 Halle (DE)
(74) Vertreter: Kaewert, Klaus
(86) Internationale Anmeldenummer: EP9601608
(87) Internationale Veröffentlichungsnummer: WO97039846

(56) Entgegenhaltungen:
- WO-A-94/22612

## Beschreibung

Die Erfindung betrifft ein Verfahren zur oberflächenschönenden, versteifenden Strukturierung dünner Materialbahnen und Folien, bei dem gekrümmte Materialbahnen und Folien, durch Stützelemente abgestützt, mit einem Über- oder Unterdruck beaufschlagt werden.

Es sind zahlreiche Verfahren bekannt, um dünnwandige Materialbahnen und Folien durch Strukturieren zu versteifen. Zu den bekannten Umformverfahren gehören das Tiefziehen, Pressen und Walzen mit Formwerkzeugen, wie z.B. Matrizen und Profilwalzen. Diese Umformverfahren ergeben den Nachteil, daß die zu strukturierenden Materialbahnen und Folien durch die Formwerkzeuge stark plastifiziert werden und daß die Oberflächengüte des Ausgangsmaterials durch die mechanische Flächenpressung leidet.

Die Europäische Patentanmeldung 0 441 618 A 1 beschreibt ein Verfahren, bei dem polyedrische, u.a. rautenförmige und sechseckige Strukturen mit Hilfe von zwei Prägewalzen erzeugt werden. Bei diesem rein mechanischen Formgebungsverfahren wird die Oberflächengüte des Ausgangswerkstoffs infolge starker mechanischer Verformung erheblich beeinträchtigt. In dem US Patent 4.576.669 wird vorgeschlagen, eine Kunststoffolie über eine Walze zu führen, in der sich Näpfchen befinden, in die die Kunststoffolie durch Vakuum hineingezogen wird. Auf diese Weise reduziert sich die Querschnittsfläche der Folie, es können Risse entstehen, und die Formsteifigkeit der gesamten Folie nimmt dadurch nicht merklich zu. Um axiale Sicken in Dosen einzuprägen, ist ferner eine Vorrichtung bekannt, mit deren Hilfe die Dose auf der Innenseite mit axialen, starren Elementen abgestützt und auf der Außenseite mit einer elastischen Rolle mit Druck beaufschlagt wird (DE 35 87 768 T 2). Die Formsteifigkeit des so mit axialen Sicken versehenen Materials ist jedoch unbefriedigend, da Sicken aus geometrischen Gründen keine mehrdimensionale Formsteifigkeit ergeben. Ein Verfahren, bei dem runde, kalottenförmige Strukturen mittels eines gelochten, zylindrischen Formwerkzeugs und einer elastischen Druckwalze in eine Folie eingedrückt werden, führt ebenfalls zu einer Reduzierung des Querschnitts der Folie und verbessert nicht oder nur ganz wenig die Formsteifigkeit, da zwischen den runden, kalottenförmigen Strukturen breite, nicht verformte Teilflächen stehen bleiben (Französische Anmeldung 1.283.530).

Ferner ist ein Verfahren bekannt, nach dem dünne Materialbahnen oder Folien beulartig strukturiert werden. Dabei wird die gekrümmte, dünne Materialbahn oder Folie auf der Innenseite durch linienförmige Stützelemente abgestützt und von außen mit Druck beaufschlagt. Der äußere Druck wird hydraulisch aufgebracht. Auf diese Weise entstehen viereckige, versetzte Beulstrukturen, die die Formsteifigkeit der Materialbahn wesentlich verbessern (Deutsche Offenlegungsschrift 25 57 215, Deutsche Patentschrift DE 43 11 978). Dieser Prozeß des Beulstrukturierens unterscheidet sich von dem in der Anmeldung 0 441 618 A 1 prinzipiell dadurch, daß nicht zwei Prägewalzen erforderlich sind, die mechanisch wirken, sondern daß nur ein Kern mit linienförmigen Stützelementen benötigt wird, auf denen die Materialbahn aufliegt, und gegen die sie hydraulisch angepreßt wird. Diese gekrümmte Materialbahn, die innen durch mit im Abstand zueinander angeordnete Stützelemente (Ringe oder schraubenförmige Spirale) abgestützt ist, wird durch den äußeren Druck instabil. Die Instabilität löst eine mehrdimensionale Faltung aus, und dadurch bilden sich in einem Selbstorganisationsprozeß viereckige, versetzte Beulstrukturen, deren Beulmulden sich frei einstellen. Da hierbei nur geringe Dehnungen und Stauchungen im Material auftreten, wird die Oberflächengüte des Ausgangswerkstoffs nicht oder nur ganz wenig beeinträchtigt. Diese viereckig, versetzt strukturierte Materialbahn (siehe Fig. 2 in DE 43 11 978) besitzt jedoch in der ebenen Gestalt keine isotrope Formsteifigkeit, da sie quer zu den durchgehenden Beulfalten biegeweich und parallel dazu formsteif ist. Eine nahezu isotrope Formsteifigkeit wird durch ein Verfahren erreicht, bei dem hexagonale Beulstrukturen (veröffentlichte internationale Patentanmeldung PCT/EP 94/01043 (= WO-A- 9422612, nächst liegonder Stand der Technik), siehe dort Abb. 5 b und 5 c) erzeugt werden. Anstelle der hydraulischen Druckaufprägung kann der Druck auch mittels eines nicht strukturierten, elastischen Kissens oder durch einen nicht strukturierten Elastomeren aufgebracht werden. Die Stützelemente, gegen die die Materialbahn gedrückt wird, bestehen aus einem starren oder auf dem Kern verschiebbaren, flexiblen Werkstoff. Die so erzeugten sechseckigen Strukturen besitzen jedoch noch keine einheitliche Form.

Alle bekannten Strukturierungsverfahren, die mechanische Formwerkzeuge verwenden, besitzen den wesentlichen Nachteil, daß das Material stark plastifiziert wird, daß die Materialbahn durch das Tiefziehen eine verminderte Wanddicke erhält und reißen kann, daß durch das Tiefziehen Zipfeiungen in der Materialbahn entstehen und daß die Oberflächengüte des Ausgangsmaterials durch die Flächenpressung der Formwerkzeuge und durch das starke Plastifizieren des Werkstoffs leidet. Bei diesen bekannten, rein mechanischen Strukturierungsverfahren tritt bei gegebener räumlicher Strukturierungstiefe ein hoher plastischer Verformungsgrad des Materials auf, der die Oberflächengüte des Ausgangswerkstoffs beeinträchtigt.

Ein weiterer, wesentlicher Nachteil der in O.S. 25 57 215, DE 43 11 978 und PCT/EP 94/01043 beschriebenen hydraulischen Strukturierungsverfahren besteht darin, daß zwar der plastische Verformungsgrad im Vergleich zu den rein mechanischen Verfahren reduziert ist und die Oberflächengüte des Ausgangsmaterials nicht beeinträchtigt wird, daß aber die erzeugten Strukturen keine gleichmäßige Form besitzen. Die Ursachen hierfür sind u. a. unvermeidbare Inhomogenitäten des Werkstoffs, die Toleranzen der Wanddicke des Ausgangswerkstoffs und eine nicht exakt gleichmäßige Druckbeaufschlagung der Materialbahn.

Die Aufgabe der vorliegenden Erfindung besteht darin, ein Verfahren anzugeben, das die Vorteile der bekannten Verfahren nutzt, ohne ihre Nachteile in Kauf zu nehmen. Die Aufgabe besteht darin, die Materialbahn in der Weise zu strukturieren, daß die Oberfläche minimal verändert wird, daß geringe plastische Verformungen des Materials auftreten und dennoch eine gleichmäßige, dreidimensionale Strukturierung stattfindet. Ein weiteres Ziel der Erfindung besteht darin, ein Verfahren anzugeben, durch das es gelingt, eine verbesserte Formsteifigkeit in der ebenen Gestalt der strukturienen Materialbahn bei minimaler Querschnittsänderung des Werkstoffs zu erzielen.

Die Lösung dieser Aufgabe besteht gemäß Anspruch 1 erfindungsgemäß darin, daß eine gekrümmte Materialbahn, die außen durch elastische, nicht strukturierte Elemente mit Druck beaufschlagt wird, gegen starre, viereckige oder sechseckige Stützelemente gedrückt wird, und daß diese Stützelemente so angeordnet werden, daß ihre Geometrien den durch Selbstorganisation sich frei einstellenden Beulfalten entsprechen. Dadurch, daß bei diesem Verfahren gemäß der Erfindung Beulstrukturen in ihre endgültige Form in die Materialbahn hineinspringen und dabei keine Berührung mit einem mechanischen Formwerkzeug, gegen das sie gepreßt werden, stattfindet, unterscheidet sich dieses Verfahren ganz grundsätzlich von allen bekannten, rein mechanischen Strukturierungsverfahren. Die verbesserte Formsteifigkeit bei der Beulstrukturierung entsteht durch die Ausbildung einer Beule, die als Kalotte ihre dreidimensionale, formsteife Form selbst einstellt. Dieser Effekt wurde bei experimentellen Untersuchungen immer wieder bestätigt. Nach dem Verfahren gemäß der Erfindung entsprechen die Geometrien der starren, viereckigen oder sechseckigen Stützelemente den sich durch Selbstorganisation einstellenden Beulfalten. Auf diese materialschonende Weise entstehen gleichmäßig angeordnete Strukturen, da durch Vorgabe der optimierten, viereckigen oder sechseckigen Stützelemente die negativen Wirkungen (u.a. Inhomogenität des Ausgangswerkstoffs, Toleranzen seiner Wanddicke und ungleichmäßige Druckbeaufschlagung) kompensiert werden und die Vorteile des oberflächenschonenden und formversteifenden Beulstrukturierungsverfahrens mit geringer Plastifizierung des Materials voll genutzt werden.

Eine Ausgestaltung des Verfahrens gemäß der Erfindung besteht darin, daß sich zwischen der zu strukturierenden Materialbahn und den Stützelementen eine zusätzliche flexible Materialschicht befindet, damit unerwünschte mechanische Abdrücke der Stützelemente in einer oberflächenempfindlichen Materialbahn abgefedert werden. Die Beulstrukturen der Materialbahn sind bei Verwendung dieser zusätzlichen flexiblen Materialschicht flacher ausgebildet als ohne diese zusätzliche flexible Materialschicht. Durch die flexible Materialschicht nach dem Verfahren gemäß der Erfindung ergibt sich ein weiterer Vorteil: Experimentelle Untersuchungen haben gezeigt, daß sich bei einer freien Ausgestaltung des Beulstrukturierens vorzugsweise sechseckförmige Strukturen bilden, wenn eine Materialbahn gegen einen Kern mit viereckigen Stützelementen gedrückt wird und zwischen der Materialbahn und den Stützelementen eine flexible Materialschicht angeordnet ist. Der technische und wirtschaftliche Vorteil dieser Technik besteht darin, daß viereckige Stützelemente einfacher und kostengünstiger herzustellen sind als sechseckige und daß Materialbahnen mit einer hochempfindlichen Oberfläche hierdurch besonders schonend zu strukturieren sind. Da die Strukturtiefe infolge der abfedernden Wirkung der flexiblen Materialschicht jedoch geringer ist als ohne Verwendung der flexiblen Materialschicht, ist die Formversteifung der Materialbahn ebenfalls geringer.

In einer weiteren Ausgestaltung der Erfindung werden beulstrukturierte Materialbahnen oder Folien in eine dreidimensionale, schalenförmige Gestalt gebracht, ohne daß hierfür komplizierte Formwerkzeuge erforderlich sind. Hierzu werden erfindungsgemäß sechseckige, beulstrukturierte Materialbahnen oder Folien dadurch mehrdimensional verformt, daß die Beulfalten der sechseckigen Strukturen tiefer gebogen werden, wenn der örtliche Krümmungsradius des Bauteils vergrößert werden soll, und die Beulfalten der Strukturen teilweise oder vollständig aufgebogen werden, wenn die örtliche Krümmung des Bauteils abgeflacht werden soll. Hierbei treten nur Biegeumformungen der Strukturfalten mit geringer Plastifizierung des Werkstoffs auf.

Die Erfindung ist anhand eines Ausführungsbeispieles in der beiliegenden Zeichnung dargestellt und wird nachfolgend näher beschrieben. Eine weitere vorteilhafte Maßnahme ergibt sich aus dem Unteranspruch.

Fig. 1 zeigt den schematischen Aufbau einer Vorrichtung zur Herstellung beulstrukturierter Materialbahnen und/oder Folien mit einer Walze, auf der Stützelemente angeordnet sind, und mit einer flexiblen Druckwalze (radialer Querschnitt).

Fig. 2 zeigt die Aufsicht auf eine Beulstruktur, die mit Hilfe einer Vorrichtung nach Fig. 1 hergestellt ist, die mit versetzten, viereckigen Stützelementen ausgestattet wurde.

Fig. 3 und Fig. 4 zeigen die Aufsicht auf zwei Beulstrukturen, die mit Hilfe einer Vorrichtung nach Fig.1 hergestellt sind, die mit mit zwei verschiedenen, sechseckigen Stützelementen ausgestattet wurde.

Fig. 5 zeigt schematisch den Aufbau einer Vorrichtung zur Herstellung von beulstrukturierten Materialbahnen mit einer Walze, auf der Stützelemente angeordnet sind, einer flexiblen Druckwalze und einer zusätzlichen flexiblen Materialschicht.

Fig. 6 zeigt schematisch den Aufbau einer Vorrichtung zur Herstellung von beulstrukturierten Dosen mit einer Walze, auf der Stützelemente angeordnet sind, und einer flexiblen Druckwalze (radialer Querschnitt).

Fig. 7 zeigt schematisch den Aufbau einer Vorrichtung zur Herstellung von beulstrukturierten Dosen mit einer Walze, auf der Stützelemente angeordnet sind (axiale Aufsicht), und einer flexiblen Druckwalze (axialer Querschnitt).

Fig. 8 und Fig. 9 zeigen schematisch einen beulstrukturierten, zylindrischen Behälter, der mit kugelförmigen Füllkörpern gefüllt ist (radialer Querschnitt in Fig. 8; axialer Querschnitt in Fig. 9).

Fig. 10 und Fig. 11 zeigen schematisch eine beulstrukturierte Materialbahn (Schiene), die zwei Knochenteile zusammenhält und stützt (axialer Querschnitt in Fig. 10; radialer Querschnitt in Fig. 11).

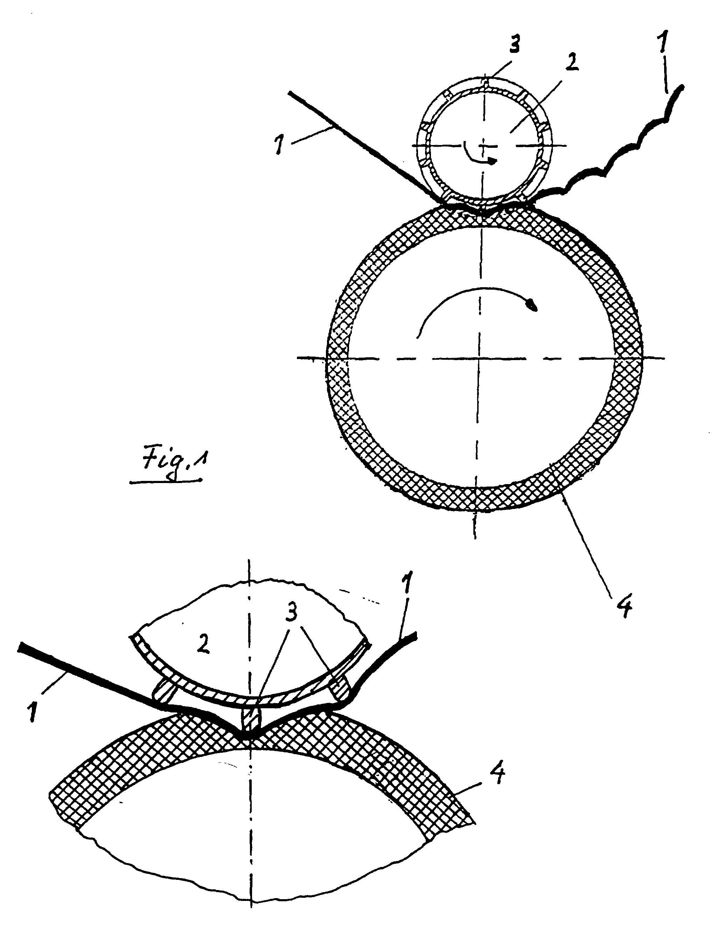

Die in der Fig. 1 dargestellte Vorrichtung zeigt den prinzipiellen Aufbau einer Vorrichtung zur Herstellung beulstrukturierter Materialbahnen oder Folien. Die Materialbahn 1 wird um die Walze 2, auf der die Stützelemente 3 angeordnet sind, geführt und durch die flexible Druckwalze 4 an die Stützelemente 3 gedrückt. Durch den von der flexiblen Druckwalze 4 auf die gekrümmte Materialbahn 1 aufgeprägten Druck kommt es zum Einbeulen der Materialbahn 1 zwischen den Stützelementen 3, wobei die Beulen in ihre endgültige Form hineinspringen, ohne im Bereich der Beulmulden eine mechanische Form zu berühren.

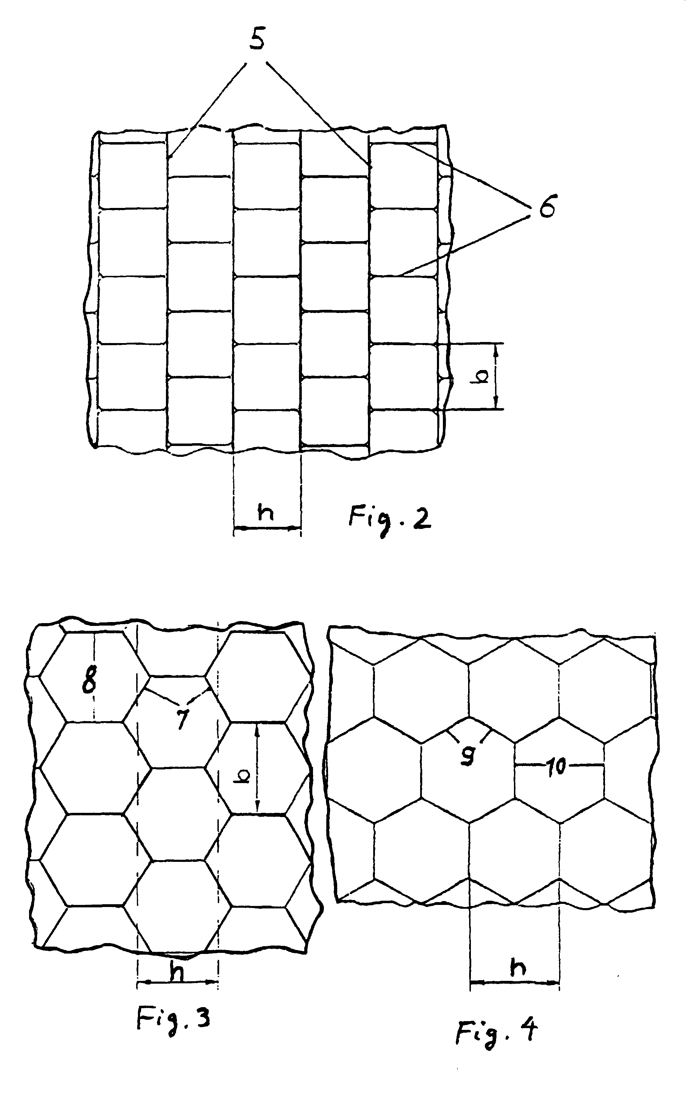

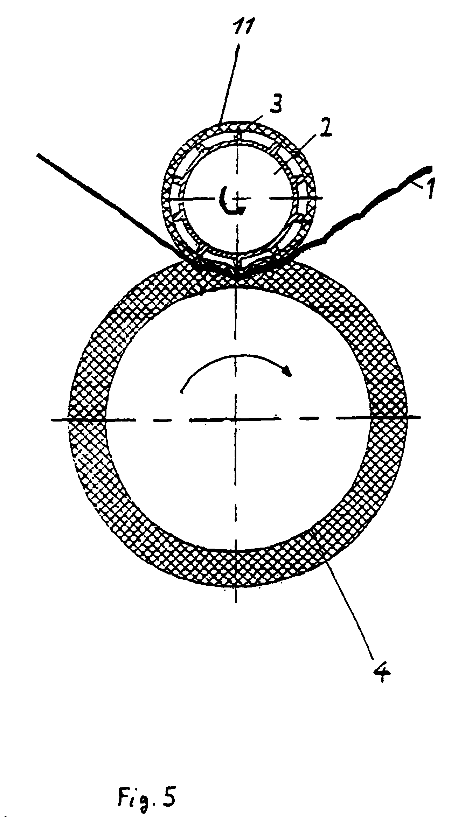

Die in der Fig. 2, 3 und 4 dargestellten Aufsicnten zeigen abgewicklete, beulstrukturierte Materialbahnen, die mit Hilfe einer Vorrichtung nach Fig. 1 hergestellt wurden. In Fig. 2 sind versetzte Vierecke dargestellt, die sich aus den Beulfalten 5 (in Transportrichtung der Materialbahn) im Abstand h zueinander und den Beulfalten 6 (quer zur Transportrichtung der Materialbahn) im Abstand b zueinander ergeben. In Fig. 3 und 4 sind die Aufsichten von zwei alternativen Anordnungen von Sechseckstrukturen dargestellt. In Fig. 3 sind die Beulfalten 7 (in Transportrichtung der Materialbahn) zick-zackförmig und haben den mittleren Abstand h zueinander. Die Beulfalten 8 (quer zur Transportrichtung der Materialbahn) haben den Abstand b zueinander. In Fig. 4 sind die Beulfalten 9 (quer zur Transportrichtung der Materialbahn) zick-zackförmig. Die Beulfalten 10 (in Transportrichtung der Materialbahn) haben den Abstand h zueinander.

Die Fig. 5 dargestellte Vorrichtung zeigt den schematischen Aufbau der Vorrichtung zur Herstellung von beulstrukturierten Materialbahnen oder Folien mit einer flexiblen Druckwalze 4 und einer Walze 2, auf der Stützelemente 3 angeordnet sind, die von einer weiteren flexiblen Materialbahn 11 umgeben sind. Hierdurch wird erreicht, daß unerwünschte mechanische Abdrücke durch denkbare Unregelmäßigkeiten der Stützkonturen 3 ausgeglichen werden.

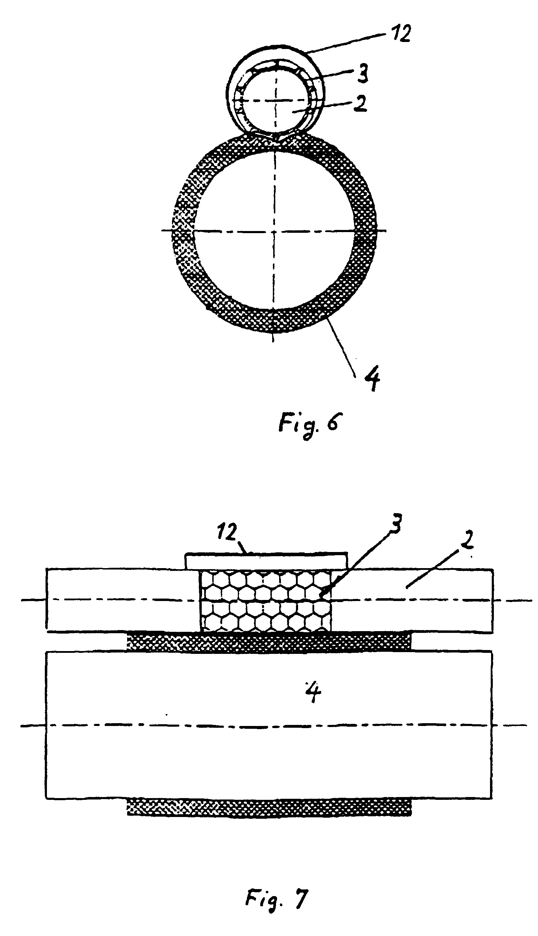

Die Darstellungen der Vorrichtungen in Fig. 6 und Fig. 7 erläutern schematisch die Herstellung von beulstrukturierten Dosen oder dünnwandigen Zylindern. Fig. 6 stellt den radialen Querschnitt der Vorrichtung mit der Walze 2, auf der die Stützelemente 3 angeordnet sind, mit der flexiblen Druckwalze 4 und mit dem zylindrischen Dosenrumpf bzw. der zylindrischen Wand 12 dar. Fig. 7 zeigt diese genannten Teile in axialer Ansicht.

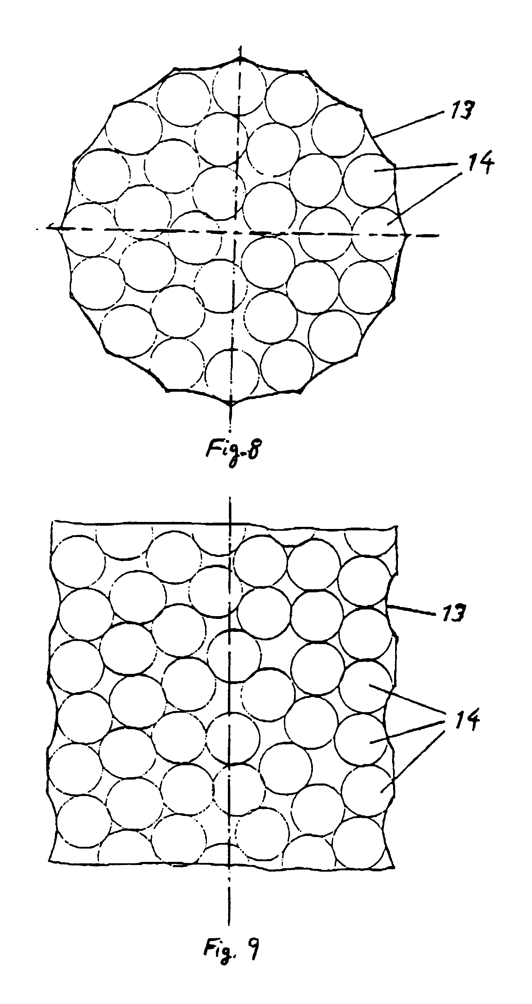

Die Fig. 8 und Fig. 9 zeigen schematisch den radialen Querschnitt (Fig. 8) und den axialen Querschnitt (Fig. 9) eines beulstrukturierten, zylindrischen Behälters 13, der mit Füllkörpern 14 gefüllt ist. Die Beulstrukturen der Behälterwand 13 sind den Rundungen der Füllkörper 14 in der Weise angepaßt, daß der freie Strömungsquerschnitt zwischen den wandnahen Füllkörpern 14 und der beulstrukturierten Behälterwand 13 gering ist.

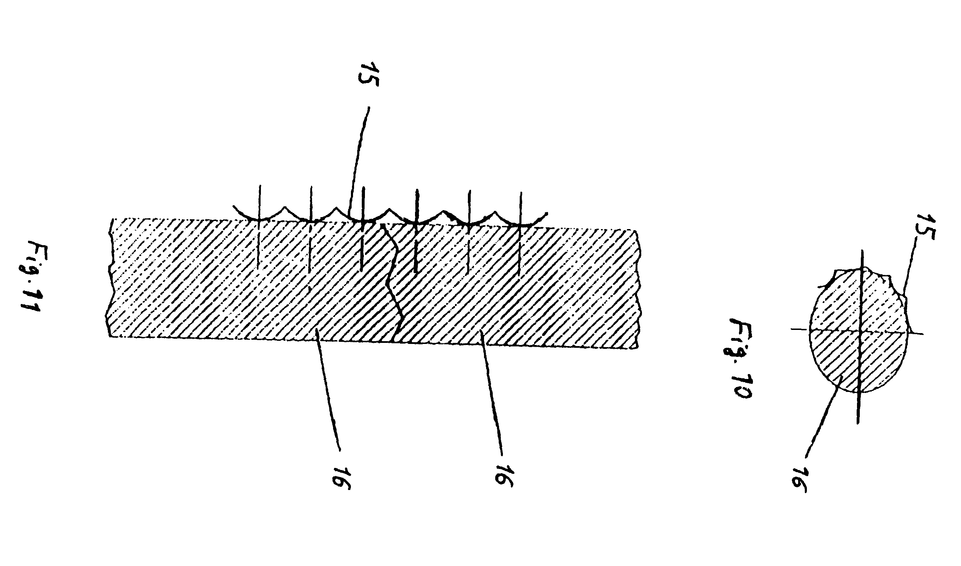

In den Fig. 10 und 11 sind der radiale Querschnitt (Fig. 10) und der axiale Querschnitt (Fig. 11) einer beulstrukturierten Materialbahn 15, die zwei Knochenteile 16 zusammenhält und schient, schematisch dargestellt. Die beulstrukturierte Materialbahn 15, die schalenförmig ausgebildet ist, ist um die beiden Knochenteile 16 gebogen und berührt die beiden Knochenteile nur mit den Beulmulden. Auf diese Weise wird die Knochenhaut, die den Knochen außen umgibt, nur an den kleinen Auflageflächen zwischen den Beulmulden und dem Knochen hoch beansprucht. Zwischen diesen Auflageflächen kann die Knochenhaut mit Blut versorgt werden und stirbt dadurch nicht ab. Ferner besitzt die beulstrukturierte, schalenförmige Schiene bessere Festigkeitseigenschaften als die bekannten glatten Schienen, da sie im Vergleich zu einer nicht strukturierten Schiene, die aus dem gleichen Werkstoff besteht und dieselben geometrischen Abmessungen hat, in radialer Richtung biegesteifer und in axialer Richtung des Knochens elastischer ist. Auf diese Weise paßt sich die beulstrukturierte Schiene den Festigkeitseigenschaften eines Knochens besser an als die bekannten glatten Schienen.

## Patentansprüche

1. Verfahren zur oberflächenschönenden, versteifenden Strukturierung dünner Materialbahnen (1) und Folien, bei dem gekrümmte Materialbahnen und Folien durch Stützelemente abgestützt und mit einem Über- oder Unterdruck beaufschlagt werden, **dadurch gekennzeichnet,**
**daß** starre viereckige oder sechseckige Stützelemente (3) zum Einsatz kommen,
**daß** die gekrümmte Materialbahn (1) von außen durch elastische nicht strukturierte Elemente (4) mit Druck beaufschlagt wird,
**daß** die Materialbahn (1) in der Krümmung mit Druck beaufschlagt wird und
die Stützelemente (3) so bemessen sind, daß ihre Geometrie den durch Selbstorganisation sich frei einstellenden Beulfalten entspricht.

2. Verfahren nach Anspruch 1, **dadurch gekennzeichnet, daß** sich zwischen den Stützelementen (3) und der zu strukturierenden Materialbahn (1) zusätzlich eine flexible Materialschicht (11) befindet.

## Claims

1. Process for profiling thin material webs (1) and sheets so as to enhance the surface and impart stiffening, in which curved material webs and sheets are supported by supporting elements and subjected to an excess or reduced pressure, **characterized in that** rigid quadangular or hexagonal supporting elements (3) are used,
**in that** the curved material web (1) is subjected to external pressure by elastic, non-profiled elements (4),
**in that** the material web (1) is subjected to pressure in the curve, and
the supporting elements (3) are so dimensioned that their geometry corresponds to the dome creases that arise freely of their own accord.

2. Process according to Claim 1, **characterized in that** in addition a flexible material layer (11) is located between the supporting elements (3) and the material web (1) to be profiled.

## Revendications

1. Procédé pour structurer en rigidifiant, tout en ménageant la surface, des bandes de matériau et des feuilles (1) minces, dans lequel des bandes de matériau et des feuilles incurvées sont soutenues par des éléments d'appui et sollicitées par une pression positive ou négative (vide), **caractérisé en ce que** l'on utilise des éléments d'appui (3) tétragonaux ou hexagonaux rigides, **en ce que** la bande de matériau (1) incurvée est sollicitée par une pression, depuis l'extérieur, au moyen d'éléments élastiques (4) non structurés, **en ce que** la bande de matériau (1) est sollicité par une pression, à l'endroit de la courbure, et les éléments d'appui (3) sont de dimensions telles que leur géométrie correspond au pliage de bosselure s'établissant librement suite à l'auto-organisation.

2. Procédé selon la revendication 1, **caractérisé en ce qu'**une couche de matériau (11) flexible se trouve en plus entre les éléments d'appui (3) et la bande de matériau (1) à structurer.
